(19) Europäisches Patentamt
European Patent Office
Office européen des brevets

(11) EP 4 070 791 A1

(12) **EUROPEAN PATENT APPLICATION**
published in accordance with Art. 153(4) EPC

(43) Date of publication:
**12.10.2022 Bulletin 2022/41**

(21) Application number: **20895206.9**

(22) Date of filing: **04.12.2020**

(51) International Patent Classification (IPC):
***A61K 31/335*** (2006.01) ***A61K 9/10*** (2006.01)
***A61K 9/12*** (2006.01) ***A61K 31/56*** (2006.01)
***A61K 31/573*** (2006.01) ***A61K 31/58*** (2006.01)
***A61K 47/02*** (2006.01) ***A61K 47/10*** (2017.01)
***A61K 47/18*** (2017.01) ***A61K 47/26*** (2006.01)
***A61K 47/32*** (2006.01) ***A61P 11/02*** (2006.01)
***A61P 37/08*** (2006.01) ***A61P 43/00*** (2006.01)

(52) Cooperative Patent Classification (CPC):
**A61K 9/10; A61K 9/12; A61K 31/335; A61K 31/56; A61K 31/573; A61K 31/58; A61K 47/02; A61K 47/10; A61K 47/18; A61K 47/26; A61K 47/32; A61P 11/02; A61P 37/08; A61P 43/00**

(86) International application number:
**PCT/JP2020/045314**

(87) International publication number:
**WO 2021/112242 (10.06.2021 Gazette 2021/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.12.2019 JP 2019221421**

(71) Applicant: **Toko Yakuhin Kogyo Co., Ltd.
Osaka-shi, Osaka 530-0022 (JP)**

(72) Inventors:
- **KAMISHITA, Taizou
Osaka-shi, Osaka 530-0022 (JP)**
- **MIYAZAKI, Takashi
Osaka-shi, Osaka 530-0022 (JP)**
- **TANAKA, Chikara
Yokohama-shi, Kanagawa 222-8567 (JP)**
- **OHSAWA, Fukuichi
Tokyo 104-8002 (JP)**

(74) Representative: **Hoffmann Eitle
Patent- und Rechtsanwälte PartmbB
Arabellastraße 30
81925 München (DE)**

# (54) PHARMACEUTICAL COMPOSITION COMPRISING STEROID COMPOUND AND OLOPATADINE

(57) The present invention relates to a homogeneous composition comprising a steroid compound and olopatadine, wherein the steroid compound is in a stable suspension state, and the olopatadine is in a stable solution state, and a process thereof.

EP 4 070 791 A1

## Description

TECHNICAL FIELD

The present invention relates to a homogeneous composition comprising a steroid compound and olopatadine, wherein the steroid compound is in a stable suspension state, and the olopatadine is in a stable solution state, and a process thereof. The present invention may be also used for treating allergic rhinitis.

BACKGROUND ART

Steroid compounds have antiinflammatory, and have been broadly used for treating inflammatory disease such as dermatitis, asthma, and rhinitis. For example, beclometasone dipropionate, fluticasone propionate, mometasone furoate, and fluticasone furoate which are a kind of steroid compounds are glucocorticoid steroid compounds which are topically used to reduce inflammation in skin or airway, and have been already commercially supplied as nasal drop for treating allergic rhinitis (Patent Reference 1).

The water-solubility of these steroid compounds are very poor, and thus it may be necessary to include an organic solvent to prepare a nasal drop for treating allergic rhinitis comprising the steroid compound. Considering that the administration site is a sensitive nasal mucosa, however, it is difficult to include a volume of irritating organic solvent to make olopatadine dissolved. Or, as another means, it might be useful to include a solubilizing agent. However, the selection of safe solubilizing agents having low mucosal irritation has a certain limitation. Eventually, an already-marketed nasal drop preparation thereof is only an aqueous suspension whose pH is a week acidic range of 4.5 - 6.5.

However, such safe suspension has some problem, and the suspension dispersibility is not so stable even if using a broadly-used suspending agent such as crystalline cellulose and carboxymethyl cellulose sodium (carmellose sodium) . Thus, it is typical to remind the user to shake the bottle before use.

Olopatadine has been already used as antihistamine in the treatment of allergic rhinitis or allergic conjunctivitis. Patent References 2 and 3 disclose a nasal drop comprising olopatadine hydrochloride, specifically an invention directed to a drug product comprising 0.54 - 0.62 % (w/v) olopatadine free base, wherein the pH is adjusted to 3.6 - 3.8, which is a topical formulation in use for treating and/or preventing nasal allergic or inflammatory disorder.

As a drug preparation based on Patent Reference 2, PATANASE™ (olopatadine hydrochloride solution for nasal use) 0.6 % is an only commercial preparation for use in nasal administration, comprising olopatadine. According to the label information, the preparation comprises olopatadine hydrochloride whose amount is 0.6 % as olopatadine free form, 0.01 % benzalkonium chloride, and non-specified amounts of disodium hydrogen phosphate, disodium edetate hydrate, sodium chloride, hydrochloric acid and/or sodium hydroxide (for adjusting the pH), and purified water, but the pH is adjusted to about 3.7 which is a very irritant pH for intranasal mucosa.

A similar composition to PATANASE™ wherein the pH is adjusted to about 7 has been also marketed under tha name PATANOL™, but the concentration of olopatadine in PATANOL™ is very low, *i.e.,* 0.1 % as olopatadine free form, because the solubility of olopatadine is very low around neutral pH.

The poor solubility of olopatadine had been studied in detail in Pantent Reference 2, which includes two kinds of "figures showing pH-solubility profile of olopatadine", said figures show that olopatadine whose concentration is 0.2 % or more as olopatadine free base is not soluble in a solution having pH of 5 or higher.

Thus, in case that a steroid compound and olopatadine are formulated as a combination drug to prepare a composition for use in nasal administration, there are some problem such as solubility and suspensibility in each ingredient, and it has been difficult to satisfy a physiologically sutable pH, a sutable concentration, and sufficient solubility and suspensibility.

PRIOR ART

[Patent Reference]

[Patent Reference 1] JP 4838493 B
[Patent Reference 2] JP 5149308 B
[Patent Reference 3] JP 6203967 B

SUMMARY OF INVENTION

(Technical Problem)

The purpose of the present invention is to provide a pharmaceutical composition of nasal drop as a combination drug of olopatadine and a steroid compound, which is in a physiologically mildly-irritating pH range of 4.0 - 7.0, preferably 4.5 - 6.5, more preferably 5.0 - 6.0, wherein the steroid compound is in stable suspension state, and olopatadine is in stable solution state, in high concentrations, in particular, an aqueous nasal drop composition for spray.

(Solution to Problem)

The present inventors have extensively studied on the above problem and have found that a steroid compound can be maintained in unprecedented very-stable suspension-dispersion state and olopatadine can be maintained in unprecedented very-stable solution state, in high concentrations even at pH range of 5.0 - 6.0 by adding carboxy vinyl polymer to a nasal drop composition comprising a steroid compound and olopatadine, said carboxy vinyl polymer is usually used as thickening agent or viscous agent, not used as solubilizer or solubilizing agent. Based upon the new findings, the present invention has been accomplished.

The present invention may provide the following embodiments.

(Item 1) A pharmaceutical composition comprising a steroid compound, and olopatadine or a pharmaceutically acceptable salt thereof.

(Item 2) The pharmaceutical composition of Item 1, wherein the olopatadine or a pharmaceutically acceptable salt thereof is olopatadine hydrochloride.

(Item 3) The pharmaceutical composition of Item 1 or 2, further comprising carboxy vinyl polymer as a thickening agent.

(Item 4) The pharmaceutical composition of any one of Items 1 - 3, comprising a steroid compound, olopatadine or a pharmaceutically acceptable salt thereof, and carboxy vinyl polymer, wherein the pH is adjusted to 4.0 - 7.0 (preferably 4.5 - 6.5, more preferably 5.0 - 6.0).

(Item 5) The pharmaceutical composition of any one of Items 1 - 4, wherein the olopatadine or a pharmaceutically acceptable salt thereof is olopatadine hydrochloride, and the olopatadine hydrochloride is in solution state in a concentration of 0.2 % (w/w) or more.

(Item 6) The pharmaceutical composition of any one of Items 1 - 5, wherein the steroid compound is in stable dispersion state in a concentration of 0.005 - 1 % (w/w).

(Item 7) The pharmaceutical composition of any one of Items 1 - 6, wherein the steroid compound is any one of beclometasone dipropionate, fluticasone propionate, mometasone furoate, or fluticasone furoate.

(Item 8) The pharmaceutical composition of Item 7, wherein the steroid compound is fluticasone furoate.

(Item 9) The pharmaceutical composition of any one of Items 1 - 8, wherein the concentration of the carboxy vinyl polymer is 0.1 - 2 % (w/w).

(Item 10) The pharmaceutical composition of any one of Items 4 - 9, wherein the pH is adjusted with sodium hydroxide and/or hydrochloric acid as a pH adjuster.

(Item 11) The pharmaceutical composition of any one of Items 4 - 10, wherein the pH is adjusted with L-arginine as a neutralizing agent.

(Item 12) The pharmaceutical composition of any one of Items 1 - 11, further comprising one or more suspension agents.

(Item 13) The pharmaceutical composition of Item 12, wherein the suspension agent comprises polysorbate 80.

(Item 14) The pharmaceutical composition of any one of Items 1 - 13, further comprising one or more preservatives.

(Item 15) The pharmaceutical composition of Item 14, wherein the preservative comprises benzalkonium chloride.

(Item 16) The pharmaceutical composition of any one of Items 1 - 15, further comprising one or more stabilizing agents.

(Item 17) The pharmaceutical composition of Item 16, wherein the stabilizing agent comprises disodium edetate hydrate.

(Item 18) The pharmaceutical composition of any one of Items 1 - 17, further one or more isotonizing agents.

(Item 19) The pharmaceutical composition of Item 18, wherein the isotonizing agent comprises sodium chloride and/or glycerin.

(Item 20) The pharmaceutical composition of Item 18 or 19, wherein the concentration of the isotonizing agent is 0.1 - 10 % (w/w).

(Item 21) The pharmaceutical composition of any one of Items 1 - 20, which is isotonic.

(Item 22) The pharmaceutical composition of any one of Items 1 - 21, wherein the viscosity is 250 - 2500 mPa·s (preferably 500 - 1500 mPa·s).

(Item 23) The pharmaceutical composition of any one of Items 1 - 22, whose mean liquid particle size is 30 - 100 μm when sprayed.

(Item 24) The pharmaceutical composition of any one of Items 21 - 23, wherein the pH adjuster is sodium hydroxide, the neutralizing agent is L-arginine, the suspension agent is polysorbate 80, the preservative is benzalkonium chloride, the stabilizing agent is disodium edetate hydrate, and the isotonizing agent is glycerin and sodium chloride.

(Item 25) The pharmaceutical composition of any one of Items 1 - 24, which is an aqueous liquid for use in nasal administration.

(Item 26) A formulation for spray-administration to nasal cavity, comprising the pharmaceutical composition of any one of Items 1 - 25.

(Item 27) A pharmaceutical composition prepared by adding carboxy vinyl polymer to make olopatadine dissolved at pH of 5.0 - 6.0 and simultaneously make an aqueous suspension comprising fluticasone furoate in a stable suspension state.

(Effect of the Invention)

According to the present invention, in a spray-type nasal drop as an aqueous suspension agent comprising olopatadine or a pharmaceutically acceptable salt thereof, and a steroid compound, olopatadine can be in soluble state, and the steroid compound can be in a very stable suspension state, and the nasal drop does not need to be shaken before use. In addition, the nasal drop of the present invention whose pH is adjusted to pH 5.0 - 6.0 which is physiologically acceptable as a nasal drop can easily stay in the nasal cavity. Thus, it is expected to bring in a sustained and effective improvement of the efficacy.

DESCRIPTION OF EMBODIMENTS

The steroid compound used herein is not limited to specific steroid compounds as long as the steroid compound has antiinflammatory, which includes, for example, beclometasone dipropionate, fluticasone propionate, mometasone furoate, and fluticasone furoate, and preferably fluticasone furoate. Fluticasone furoate is 6α,9-difluoro-17β-[(fluoromethylsulfanyl)carbonyl]-11β-hydroxy-16α-methyl-3-oxoandrosta-1,4-dien-17α-yl furan-2-carboxylate of the following structure, which is broadly used for treating allergic rhinitis. The concentration of a steroid compound in the present preparation is 0.005 - 1 % (w/w), preferably 0.01 - 0.1 %.

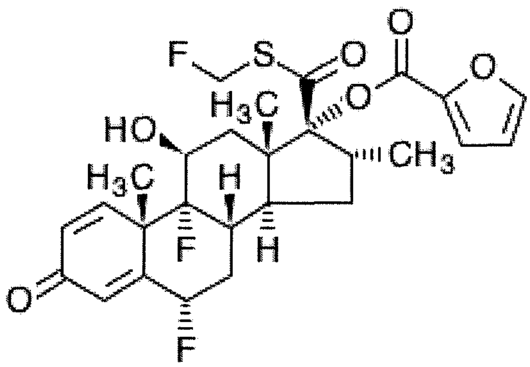

Olopatadine is a compound of formula:

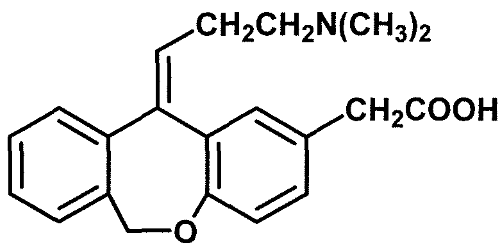

which has been already used as antihistamine for treating allergic rhinitis or allergic conjunctivitis.

Olopatadine or a salt thereof is used in the preparation of a composition for use in nasal administration, preferably used as olopatadine hydrochloride. The concentration of olopatadine in the present formulation is 0.2 % (w/w) or more, preferably 0.4 - 0.8 % (w/w), more preferably 0.4 - 0.7 % (w/w) as olopatadine hydrochloride.

The "pharmaceutically acceptable salt" is not limited to a specific one as long as the salt does not negatively affect humans or animals such as toxicity. It includes hydrochloride, citrate, succinate, hydrobromide, ascorbate, furoate, sulfate, acetate, valerate, oleinate, palmitate, laurate, stearate, bisulfate, borate, benzoate, lactate, phosphate, methanesulfonate, p-toluenesulfonate, oxalate, maleate, fumarate, tartrate, glucoheptonate, lactobionate, and laurylsulfate, as an acid-addition salt; and alkali metal salt such as sodium and potassium, and alkali-earth metal salt such as calcium and magnesium, as a basic salt.

The pharmaceutical composition of the present invention can be formulated for oral administration, parenteral administration, or topical administration, in particular, for topical administration.

The topical administration used in the present invention includes swabbing, insufflation, and inhalation. The formulation for topical administration includes, for example, ointment, lotion, cream, gel, spray, aerosol, suppository, eye drop, ear drop, nasal drop, and formulation for spray-administration to nasal cavity.

In particular, the composition for use in nasal topical administration includes nasal drop and formulation for spray-administration to nasal cavity. The "composition for use in nasal administration" means an aqueous liquid for use in nasal administration, which is in suspension state.

Carboxy vinyl polymer used herein should not be limited as long as it is what is usually used in a medical formulation. Preferably, it is carboxy vinyl polymer whose viscosity is adjusted by adding an outside shearing force. The method of the adjustment and the effect of the modified carboxy vinyl polymer are disclosed in WO 2007/123193. For example, the outside shearing force may be added with a known device giving a shearing force such as a high-speed spinning-type emulsifying device, a colloidal mill-type emulsifying device, a high-pressure emulsifying device, a roll mill-type emulsifying device, an ultrasonic-type emulsifying device and a membrane-type emulsifying device. Especially, a homo mixer-type, a comb-type, and an intermittently-jet-stream-generating-type, high-speed spinning-type emulsifying devices are preferable. The content of carboxy vinyl polymer is 0.1 to 2 % (w/w), preferably 0.25 to 1.0 %.

The suspension agent used herein includes polysorbate 80, polyoxyl 40 stearate, and/or polyoxyethylene hydrogenated castor oil 60, preferably polysorbate 80. The content of the suspension agent is 0.01 - 1 % (w/w), preferably 0.025 - 0.5 %.

The preservative used herein includes, for example, benzalkonium chloride, benzethonium chloride, and chlorobutanol, preferably benzalkonium chloride. The content of each preservative is 0.005 - 1 % (w/w), preferably 0.01 - 0.1 %.

The stabilizing agent used herein includes disodium edetate hydrate. The content of each stabilizing agent is 0.005 - 1 % (w/w), preferably 0.01 - 0.1 %.

The present aqueous composition for use in nasal administration is isotonic or around isotonic. The isotonicity may be adjusted with an isotonizing agent such as sodium chloride, boric acid, glycerin and/or glucose. The content of each isotonizing agent is 0.1 to 10 % (w/w), preferably 0.1 to 1.0 %.

The pH of the present aqueous composition for use in nasal administration needs to be adjusted to 4.0 - 7.0 (preferably 4.5 - 6.5, more preferably 5.0 - 6.0), and the adjustment of pH may be done with a pH adjuster such as sodium hydroxide, potassium hydroxide, and hydrochloric acid, preferably with sodium hydroxide.

The pH may be also adjusted with L-arginine as a neutralizing agent.

The viscosity of the present aqueous composition for use in nasal administration is generally 250 - 2500 mPa·s, preferably 500 - 1500 mPa·s.

The "solution state" in the present invention means a state wherein an objective pharmaceutical ingredient is completely dissolved, and the "dispersion state" means a state wherein an objective pharmaceutical ingredient is homogeneously suspended without depositing crystal.

The liquid particle size of the present aqueous composition for use in nasal administration means the particle size of the sprayed liquid particle. The mean liquid particle size is preferably 30 - 100 $\mu$m, more preferably 40 - 80 $\mu$m.

The "for use in nasal administration" or "as nasal drop" means a subject to be administered into the nasal cavity with a device such as a spray device. And, the "composition for use in nasal administration" means a liquid preparation to be administered by spraying the composition into nasal cavity.

The nasal-spray preparation for intranasal administration of the present invention is directed to the nasal-spray preparation in a normal nasal spray container, or in an upper-pressure-relief airless-type spray container disclosed in WO 2007/123193 and WO 2007/123207.

EXAMPLES

Hereinafter, the present invention is illustrated based on Examples, Reference examples, and Stability test, but are not limited thereto. The evaluations of the Examples and Reference examples prepared below, and Stability test was carried out according to Japanese Pharmacopoeia, unless otherwise indicated.

The viscosity measurement was carried out according to Japanese Pharmacopoeia/General Tests/Viscosity Determination/Viscosity measurement by rotational viscometer, and the details are as follows.

(Measuring method)

1.1 mL of the test sample (test preparation) was charged into a sample cup of a cone-flat plate-type rotational viscometer (cone plate type) which was beforehand set for 20°C, while being careful not to put air bubble. The sample was let stand for 5 minutes, and then subjected to a shearing force for 3 minutes. Subsequently, the viscosity of the sample was measured according to the following condition.

(Measuring condition)

Apparatus: TOKI SANGYO CO., LTD. TVE-25 type viscosity meter
Measuring range: R (full-scale torque 1437.4 μN·m)
Shearing rate: 9.575 $s^{-1}$ (2.5 rotations per minute)
Rotor: 1°34'×R24

The liquid particle size (mean liquid particle size, 10 to 100 μm (%)) was measured with a laser diffraction/scattering particle-size-distribution analyzer.

(Measuring condition)

Apparatus: Malvern SprayTec
Reading distance: 30 mm
Spray angle: 40°
Extrusion speed: 100 mm/s

The observation of a crystal was measured with a digital microscope.

(Measuring condition)

Apparatus: Digital Microscope VHX-7000 (KEYENCE CORPORATION)
Magnification: 500 x

Example 1

(Production composition)

| Ingredients | Amount (% by weight) |
|---|---|
| fluticasone furoate | 0.0275 |
| olopatadine hydrochloride | 0.443 |
| carboxy vinyl polymer | 0.50 |
| L-arginine | 0.84 |
| polysorbate 80 | 0.1 |
| disodium edetate hydrate | 0.05 |
| benzalkonium chloride | 0.01 |

(continued)

| Ingredients | Amount (% by weight) |
|---|---|
| concentrated glycerin | 0.35 |
| ethanol | 0.8 |
| sodium hydroxide | q.s. |
| hydrochloric acid | q.s. |
| purified water | q.s. (until reaching 100 %) |
| Total | 100.0 |

(Production method)

A solution of L-arginine, disodium edetate hydrate, and olopatadine hydrochloride in purified water was charged into a vacuum mixer, then a solution of benzalkonium chloride and half the amount of polysorbate 80 in purified water was added thereto, and the mixture was stirred. Separately, carboxy vinyl polymer was dissolved in purified water with stirring and the solution was added to the mixture in the vacuum mixer. The mixture was stirred in the vacuum mixer. Separately, to fluticasone furoate wetted with concentrated glycerin were added the remaining half of polysorbate 80 and purified water, and the mixture was sufficiently stirred. The mixture was added to the above mixture in the vacuum mixer, and the obtained mixture was stirred in the vacuum mixer. The pH of the mixture was adjusted to pH 5.5 optionally by adding aqueous sodium hydroxide or diluted hydrochloric acid, and then the mixture was subjected to a high-speed shearing force to adjust the viscosity to 1000 mPa·s with stirring to give a uniform nasal composition.

(Evaluation result)

The evaluation results of the obtained nasal preparation are shown below.

| | |
|---|---|
| Aspect | A viscous liquid with a white suspension, which is almost odorless |
| pH | 5.5 |
| viscosity (mPa·s) | 1005 |
| osmolality (mOs/L) | 281 |
| Mean liquid particle size (μm) | 65.5 |
| Liquid particle size of 10 to 100 μm (%) | 91.3 |

Example 2

(Production composition)

| Ingredients | Amount (% by weight) |
|---|---|
| fluticasone furoate | 0.05 |
| olopatadine hydrochloride | 0.443 |
| carboxy vinyl polymer | 0.50 |
| L-arginine | 0.73 |
| polysorbate 80 | 0.05 |
| disodium edetate hydrate | 0.05 |
| benzalkonium chloride | 0.02 |
| concentrated glycerin | 0.50 |

(continued)

| Ingredients | Amount (% by weight) |
|---|---|
| sodium hydroxide | q.s. |
| hydrochloric acid | q.s. |
| purified water | q.s. (until reaching 100 %) |
| Total | 100.0 |

(Production method)

A solution of L-arginine, disodium edetate hydrate, and olopatadine hydrochloride in purified water was charged into a vacuum mixer, then a solution of benzalkonium chloride and half the amount of polysorbate 80 in purified water was added thereto, and the mixture was stirred. Separately, carboxy vinyl polymer was dissolved in purified water with stirring and the solution was added to the mixture in the vacuum mixer. The mixture was stirred in the vacuum mixer. Separately, to fluticasone furoate wetted with concentrated glycerin were added the remaining half of polysorbate 80 and purified water, and the mixture was sufficiently stirred. The mixture was added to the above mixture in the vacuum mixer, and the obtained mixture was stirred in the vacuum mixer. The pH of the mixture was adjusted to pH 5.5 optionally by adding aqueous sodium hydroxide or diluted hydrochloric acid, and then the mixture was subjected to a high-speed shearing force to adjust the viscosity to 1250 mPa·s with stirring to give a uniform nasal composition.

(Evaluation result)

The evaluation results of the obtained nasal preparation are shown below.

| Aspect | A viscous liquid with a white suspension, which is almost odorless |
|---|---|
| pH | 5.5 |
| viscosity (mPa·s) | 1250 |
| osmolality (mOs/L) | 288 |
| Mean liquid particle size (μm) | 78.3 |
| Liquid particle size of 10 to 100 μm (%) | 80.3 |

Example 3

(Production composition)

| Ingredients | Amount (% by weight) |
|---|---|
| fluticasone furoate | 0.0275 |
| olopatadine hydrochloride | 0.665 |
| carboxy vinyl polymer | 0.50 |
| L-arginine | 0.84 |
| polysorbate 80 | 0.1 |
| disodium edetate hydrate | 0.05 |
| benzalkonium chloride | 0.01 |
| concentrated glycerin | 0.35 |
| ethanol | 0.8 |
| sodium hydroxide | q.s. |

(continued)

| Ingredients | Amount (% by weight) |
|---|---|
| hydrochloric acid | q.s. |
| purified water | q.s. (until reaching 100 %) |
| Total | 100.0 |

(Production method)

A solution of L-arginine, disodium edetate hydrate, and olopatadine hydrochloride in purified water was charged into a vacuum mixer, then a solution of benzalkonium chloride and half the amount of polysorbate 80 in purified water was added thereto, and the mixture was stirred. Separately, carboxy vinyl polymer was dissolved in purified water with stirring and the solution was added to the mixture in the vacuum mixer. The mixture was stirred in the vacuum mixer. Separately, to fluticasone furoate wetted with concentrated glycerin were added the remaining half of polysorbate 80 and purified water, and the mixture was sufficiently stirred. The mixture was added to the above mixture in the vacuum mixer, and the obtained mixture was stirred in the vacuum mixer. The pH of the mixture was adjusted to pH 5.5 optionally by adding aqueous sodium hydroxide or diluted hydrochloric acid, and then the mixture was subjected to a high-speed shearing force to adjust the viscosity to 1000 mPa·s with stirring. Finally, ethanol was added to the mixture and the mixture was stirred to give a uniform nasal composition.

(Evaluation result)

The evaluation results of the obtained nasal preparation are shown below.

| | |
|---|---|
| Aspect | A viscous liquid with a white suspension, which is almost odorless |
| pH | 5.5 |
| viscosity (mPa·s) | 1015 |
| osmolality (mOs/L) | 285 |
| Mean liquid particle size (μm) | 66.5 |
| Liquid particle size of 10 to 100 μm (%) | 89.5 |

Example 4

(Production composition)

| Ingredients | Amount (% by weight) |
|---|---|
| beclometasone dipropionate | 0.1 |
| olopatadine hydrochloride | 0.443 |
| carboxy vinyl polymer | 0.55 |
| L-arginine | 0.85 |
| polysorbate 80 | 0.1 |
| disodium edetate hydrate | 0.05 |
| benzalkonium chloride | 0.01 |
| concentrated glycerin | 0.50 |
| sodium hydroxide | q.s. |
| hydrochloric acid | q.s. |

(continued)

| Ingredients | Amount (% by weight) |
|---|---|
| purified water | q.s. (until reaching 100 %) |
| Total | 100.0 |

(Production method)

A solution of L-arginine, disodium edetate hydrate, and olopatadine hydrochloride in purified water was charged into a vacuum mixer, then a solution of benzalkonium chloride and half the amount of polysorbate 80 in purified water was added thereto, and the mixture was stirred. Separately, carboxy vinyl polymer was dissolved in purified water with stirring and the solution was added to the mixture in the vacuum mixer. The mixture was stirred in the vacuum mixer. Separately, to beclometasone dipropionate wetted with concentrated glycerin were added the remaining half of polysorbate 80 and purified water, and the mixture was sufficiently stirred. The mixture was added to the above mixture in the vacuum mixer, and the obtained mixture was stirred in the vacuum mixer. The pH of the mixture was adjusted to pH 6.0 optionally by adding aqueous sodium hydroxide or diluted hydrochloric acid, and then the mixture was subjected to a high-speed shearing force to adjust the viscosity to 1500 mPa·s with stirring to give a uniform nasal composition.

(Evaluation result)

The evaluation results of the obtained nasal preparation are shown below.

| Aspect | A viscous liquid with a white suspension, which is almost odorless |
|---|---|
| pH | 6.0 |
| viscosity (mPa·s) | 1500 |
| osmolality (mOs/L) | 279 |
| Mean liquid particle size (μm) | 75.3 |
| Liquid particle size of 10 to 100 μm (%) | 82.2 |

Example 5

(Production composition)

| Ingredients | Amount (% by weight) |
|---|---|
| fluticasone propionate | 0.05 |
| olopatadine hydrochloride | 0.665 |
| carboxy vinyl polymer | 0.50 |
| L-arginine | 0.75 |
| polysorbate 80 | 0.05 |
| disodium edetate hydrate | 0.05 |
| benzalkonium chloride | 0.01 |
| concentrated glycerin | 0.55 |
| ethanol | 0.8 |
| sodium hydroxide | q.s. |
| hydrochloric acid | q.s. |
| purified water | q.s. (until reaching 100 %) |

(continued)

| Ingredients | Amount (% by weight) |
|---|---|
| Total | 100.0 |

(Production method)

A solution of L-arginine, disodium edetate hydrate, and olopatadine hydrochloride in purified water was charged into a vacuum mixer, then a solution of benzalkonium chloride and half the amount of polysorbate 80 in purified water was added thereto, and the mixture was stirred. Separately, carboxy vinyl polymer was dissolved in purified water with stirring and the solution was added to the mixture in the vacuum mixer. The mixture was stirred in the vacuum mixer. Separately, to fluticasone furoate wetted with concentrated glycerin were added the remaining half of polysorbate 80 and purified water, and the mixture was sufficiently stirred. The mixture was added to the above mixture in the vacuum mixer, and the obtained mixture was stirred in the vacuum mixer. The pH of the mixture was adjusted to pH 5.0 optionally by adding aqueous sodium hydroxide or diluted hydrochloric acid, and then the mixture was subjected to a high-speed shearing force to adjust the viscosity to 1000 mPa·s with stirring. Finally, ethanol was added to the mixture and the mixture was stirred to give a uniform nasal composition.

(Evaluation result)

The evaluation results of the obtained nasal preparation are shown below.

| Aspect | A viscous liquid with a white suspension, which is almost odorless |
|---|---|
| pH | 5.0 |
| viscosity (mPa·s) | 1000 |
| osmolality (mOs/L) | 281 |
| Mean liquid particle size (μm) | 69.0 |
| Liquid particle size of 10 to 100 μm (%) | 87.6 |

<u>Example 6</u>

(Production composition)

| Ingredients | Amount (% by weight) |
|---|---|
| mometasone furoate | 0.05 |
| olopatadine hydrochloride | 0.665 |
| carboxy vinyl polymer | 0.50 |
| L-arginine | 0.84 |
| polysorbate 80 | 0.10 |
| disodium edetate hydrate | 0.05 |
| benzalkonium chloride | 0.01 |
| concentrated glycerin | 0.35 |
| ethanol | 0.8 |
| sodium hydroxide | q.s. |
| hydrochloric acid | q.s. |
| purified water | q.s. (until reaching 100 %) |

(continued)

| Ingredients | Amount (% by weight) |
|---|---|
| Total | 100.0 |

(Production method)

A solution of L-arginine, disodium edetate hydrate, and olopatadine hydrochloride in purified water was charged into a vacuum mixer, then a solution of benzalkonium chloride and half the amount of polysorbate 80 in purified water was added thereto, and the mixture was stirred. Separately, carboxy vinyl polymer was dissolved in purified water with stirring and the solution was added to the mixture in the vacuum mixer. The mixture was stirred in the vacuum mixer. Separately, to mometasone furoate wetted with concentrated glycerin were added the remaining half of polysorbate 80 and purified water, and the mixture was sufficiently stirred. The mixture was added to the above mixture in the vacuum mixer, and the obtained mixture was stirred in the vacuum mixer. The pH of the mixture was adjusted to pH 5.0 optionally by adding aqueous sodium hydroxide or diluted hydrochloric acid, and then the mixture was subjected to a high-speed shearing force to adjust the viscosity to 1000 mPa·s with stirring. Finally, ethanol was added to the mixture and the mixture was stirred to give a uniform nasal composition.

(Evaluation result)

The evaluation results of the obtained nasal preparation are shown below.

| Aspect | A viscous liquid with a white suspension, which is almost odorless |
|---|---|
| pH | 5.0 |
| viscosity (mPa·s) | 1000 |
| osmolality (mOs/L) | 285 |
| Mean liquid particle size (μm) | 61.0 |
| Liquid particle size of 10 to 100 μm (%) | 89.4 |

Reference example 1 (Example 1 in JP 4838493 B)

(Production composition)

| Ingredients | Amount (% by weight) |
|---|---|
| fluticasone furoate | 0.05 |
| polysorbate 80 | 0.025 |
| Avicel RC591* | 1.5 |
| glucose | 5.0 |
| disodium edetate hydrate | 0.015 |
| benzalkonium chloride | 0.015 |
| hydrochloric acid | q.s. |
| purified water | q.s. (until reaching 100 %) |
| Total | 100.0 |
| *: a mixture of crystalline cellulose and carboxymethyl cellulose sodium | |

(Production method)

In a solution of glucose in purified water was dissolved disodium edetate hydrate. Avicel RC591 was added to the solution with stirring. The Avicel RC591 was hydrated to give Suspension A. Separately, polysorbate 80 was dissolved in purified water at 50 - 60°C, and fluticasone furoate was wetted with the solution. The remaining half of polysorbate 80 and purified water was added thereto to give Suspension B. Suspension A and Suspension B were mixed and stirred. A solution of benzalkonium chloride in purified water was added to the mixed suspension, and the mixture was stirred. The pH of the mixture was adjusted to pH 6.0 with 1 N hydrochloric acid, and then the purified water was added thereto to adjust the total weight.

(Evaluation result)

The evaluation results of the obtained nasal preparation are shown below.

| Aspect | A white opaque suspension |
|---|---|
| pH | 6.0 |
| viscosity (mPa·s) | 35 |
| osmolality (mOs/L) | 278 |
| Mean liquid particle size (μm) | 72.4 |
| Liquid particle size of 10 to 100 μm (%) | 84.8 |

Reference example 2

(Example 1 in JP 4838493 B which additionally comprises olopatadine hydrochloride)

(Production composition)

| Ingredients | Amount (% by weight) |
|---|---|
| fluticasone furoate | 0.05 |
| olopatadine hydrochloride | 0.443 |
| polysorbate 80 | 0.025 |
| Avicel RC591* | 1.5 |
| glucose | 5.0 |
| disodium edetate hydrate | 0.015 |
| benzalkonium chloride | 0.015 |
| hydrochloric acid | q.s. |
| purified water | q.s. (until reaching 100 %) |
| Total | 100.0 |
| *: a mixture of crystalline cellulose and carboxymethyl cellulose sodium | |

(Production method)

In a solution of glucose in purified water were dissolved disodium edetate hydrate and olopatadine hydrochloride. Avicel RC591 was added to the solution with stirring. The Avicel RC591 was hydrated to give Suspension A. Separately, polysorbate 80 was dissolved in purified water at 50 - 60°C, and fluticasone furoate was wetted with the solution. The remaining half of polysorbate 80 and purified water was added thereto to give Suspension B. Suspension A and Suspension B were mixed and stirred. A solution of benzalkonium chloride in purified water was added to the mixed suspension, and the mixture was stirred. The pH of the mixture was adjusted to pH 6.0 with 1 N hydrochloric acid, and then the purified water was added thereto to adjust the total weight.

(Evaluation result)

The evaluation results of the obtained nasal preparation are shown below.

| | |
|---|---|
| Aspect | A white opaque suspension |
| pH | 6.0 |
| viscosity (mPa·s) | 41 |
| osmolality (mOs/L) | 313 |
| Mean liquid particle size (μm) | 94.8 |
| Liquid particle size of 10 to 100 μm (%) | 58.1 |

Reference example 3 (Example 1 in JP 5149308 B)

(Production composition)

| Ingredients | Amount (% by weight) |
|---|---|
| olopatadine hydrochloride | 0.665 |
| benzalkonium chloride | 0.01 |
| disodium edetate hydrate | 0.01 |
| sodium chloride | 0.41 |
| disodium hydrogen phosphate | 0.5 |
| anhydrous | |
| sodium hydroxide | q.s. |
| hydrochloric acid | q.s. |
| purified water | q.s. (until reaching 100 %) |
| Total | 100.0 |

(Production method)

To purified water were added disodium hydrogen phosphate anhydrous, sodium chloride, disodium edetate hydrate, benzalkonium chloride, and olopatadine hydrochloride, with stirring. In order to dissolve each ingredient, a moderate amount of diluted hydrochloric acid was added thereto for a suitable time. Purified water was added thereto, the pH was measured, the pH was adjusted to pH 3.7 optionally by adding hydrochloric acid or sodium hydroxide, and then purified water was added thereto to adjust the total weight.

(Evaluation result)

The evaluation results of the obtained nasal preparation are shown below.

| | |
|---|---|
| Aspect | A colorless and clear liquid |
| pH | 3.7 |
| viscosity (mPa·s) | 3 |
| osmolality (mOs/L) | 278 |

(continued)

| Aspect | A colorless and clear liquid |
|---|---|
| Mean liquid particle size (μm) | 51.8 |
| Liquid particle size of 10 to 100 μm (%) | 91.4 |

Examples 7 - 8, Reference examples 4 - 7

In a similar manner to Examples 1 - 6 and Reference examples 1 - 3, the following nasal compositions were prepared.

| Ingredient \ Example/Reference example | Example 7 | Example 8 | Reference example 4 | Reference example 5 | Reference example 6 | Reference example 7 |
|---|---|---|---|---|---|---|
| fluticasone furoate | 0.0275 | – | 0.0275 | 0.0275 | 0.0275 | – |
| mometasone furoate | – | 0.05 | – | – | – | 0.05 |
| olopatadine hydrochloride | 0.665 | 0.665 | 0.665 | 0.665 | 0.665 | 0.665 |
| carboxy vinyl polymer | 0.50 | 0.50 | – | – | – | – |
| Avicel RC591 | – | – | 1.5 | – | – | 1.5 |
| hydroxypropylmethylcellulose 2910 | – | – | – | 0.55 | – | – |
| polyvinyl alcohol | – | – | – | – | 2.00 | – |
| L-arginine | 0.84 | 0.84 | – | – | – | – |
| polysorbate 80 | 0.1 | 0.1 | 0.1 | 0.1 | 0.1 | 0.1 |
| disodium edetate hydrate | 0.05 | 0.05 | 0.05 | 0.05 | 0.05 | 0.05 |
| benzalkonium chloride | 0.01 | 0.01 | 0.01 | 0.01 | 0.01 | 0.01 |
| concentrated glycerin | 0.35 | 0.35 | 0.35 | 0.35 | 0.35 | 0.35 |
| sodium hydroxide | q.s. | adjusting pH to 5.5 | | | | |
| hydrochloric acid | q.s. | | | | | |
| purified water | q.s. (until reaching 100 %) | | | | | |

(Evaluation result)

The evaluation results of the obtained nasal preparations are shown below.

| | Example 1 | Example 2 | Example 3 | Example 4 | Example 5 | Example 6 | Example 7 | Example 8 |
|---|---|---|---|---|---|---|---|---|
| pH | 5.5 | 5.5 | 5.5 | 6.0 | 5.0 | 5.0 | 5.5 | 5.5 |
| viscosity (mPa·s) | 1005 | 1250 | 1015 | 1500 | 1000 | 1000 | 1000 | 1000 |
| Spray test (Aspect) | good (fine fog) | good (fine fog) | good (fine fog) | good (fine fog) | good (fine fog) | good (fine fog) | good (fine fog) | good (fine fog) |
| Mean liquid particle size (μm) | 65.5 | 78.3 | 66.5 | 75.8 | 69.3 | 61.0 | 75.2 | 76.9 |
| Liquid particle size of 10 to 100 μm (%) | 91.3 | 80.3 | 89.5 | 82.2 | 87.6 | 89.4 | 86.3 | 88.4 |

| | Reference example 1 | Reference example 2 | Reference example 3 | Reference example 4 | Reference example 5 | Reference example 6 | Reference example 7 |
|---|---|---|---|---|---|---|---|
| pH | 6.0 | 6.0 | 3.7 | 5.5 | 5.5 | 5.5 | 5.5 |
| viscosity (mPa·s) | 35 | 41 | 3 | 32 | 45 | 7 | 35 |
| Spray test (Aspect) | good (fine fog) | good (fine fog) | good (fine fog) | big liquid particle fog form | bad (jet form) | big liquid particle | big liquid particle |
| Mean liquid particle size (μm) | 72.4 | 94.8 | 51.8 | 92.7 | 336 | 98.7 | 99.1 |
| Liquid particle size of 10 to 100 μm (%) | 84.8 | 58.1 | 91.4 | 61.7 | 18.9 | 56.4 | 65.8 |

<u>Solubility-stability test of olopatadine</u>

Liquid sample for comparative test

(1) Patanase™ (Lot No.: 7FPS1A)(Concentration pH 3.8)
The content liquid was taken out of the product container, and put into a glass container to be a sample.
(2) A liquid prepared by dissolving olopatadine hydrochloride in purified water, adjusting the concentration of olopatadine to 0.6 % (w/w), and then adjusting pH to 3.8 with L-arginine (stored in a glass container)
(3) A liquid prepared by dissolving olopatadine hydrochloride in purified water, adjusting the concentration of olopatadine to 0.6 % (w/w), and then adjusting pH to 3.8 with sodium hydroxide (stored in a glass container)
(4) A liquid prepared by dissolving olopatadine hydrochloride in purified water, adjusting the concentration of olopatadine to 0.6 % (w/w), and then adjusting pH to 5.5 with L-arginine (stored in a glass container)
(5) A liquid prepared by dissolving olopatadine hydrochloride in purified water, adjusting the concentration of olopatadine to 0.6 % (w/w), and then adjusting pH to 5.5 with sodium hydroxide (stored in a glass container)

(Evaluation method)

Each sample of (1) - (5), as well as Examples 1 - 8, and Reference examples 1 - 7 were put into a glass container to be each sample for solubility stability test.

(i) Observe the sample on gross. Identify large olopatadine crystals that are different from finely dispersed suspended crystals of the steroid. If it is difficult to judge, heat the sample. When the crystal is changed about dissolution, etc., judge that the crystal is olopatadine crystal.

◦: which means that it was in clear solution state in case of (1) - (5) and Reference example 3 which do not comprise a steroid compound, or it was in homogeneous suspension state without any precipitated crystal in case of Examples 1 - 8 and Reference examples 1 - 2, and 4 - 7 which comprise the steroid compound.
×: which means that a precipitated crystal was observed.

(ii) When checking at 500 times using a digital microscope (VHX-7000), check if there are precipitated crystals of 50 μm or more other than the steroid. If it is difficult to judge whether a precipitated crystal is olopatadine crystal or not due to crystalline cellulose·carboxymethyl cellulose sodium, heat the sample. When the crystal is changed about dissolution, etc., judge that the crystal is olopatadine crystal.

◎: which means that any crystal other than the steroid crystal was not observed, there was no precipitated crystal, and it was judged that olopatadine is dissolved.
♦: which means that a precipitated crystal of 50 μm or more other than the steroid was observed, and it is judged that olopatadine was precipitated.

(Result)

| | Concentration of olopatadine | pH | Storage condition | | | | | |
|---|---|---|---|---|---|---|---|---|
| | | | Shortly after preparation | 1 day after preparation | 1 month later at room temperature | 3 months later at room temperature | 1 month later at 5 - 10°C | 3 months later at 5 - 10°C |
| (1) | 0.6 % | 3.8 | shortly after taking out<br>○<br>◎ | ○<br>◎ | ○<br>◎ | ○<br>◎ | ○<br>◎ | ○<br>◎ |
| (2) | 0.6 % | 3.8 | ○<br>◎ | ○<br>◎ | ○<br>◎ | ○<br>◎ | ○<br>◎ | ○<br>◎ |
| (3) | 0.6 % | 3.8 | ○<br>◎ | ○<br>◎ | ○<br>◎ | ○<br>◎ | ○<br>◎ | ○<br>◎ |
| (4) | 0.6 % | 5.5 | ○<br>◎ | ×<br>◆ | ×<br>◆ | ×<br>◆ | ×<br>◆ | ×<br>◆ |
| (5) | 0.6 % | 5.5 | ○<br>◎ | ×<br>◆ | ×<br>◆ | ×<br>◆ | ×<br>◆ | ×<br>◆ |
| Example 1 | 0.6 % | 5.5 | ○<br>◎ | ○<br>◎ | ○<br>◎ | ○<br>◎ | ○<br>◎ | ○<br>◎ |
| Example 2 | 0.4 % | 5.5 | ○<br>◎ | ○<br>◎ | ○<br>◎ | ○<br>◎ | ○<br>◎ | ○<br>◎ |
| Example 3 | 0.6 % | 5.5 | ○<br>◎ | ○<br>◎ | ○<br>◎ | ○<br>◎ | ○<br>◎ | ○<br>◎ |
| Example 4 | 0.4 % | 6.0 | ○<br>◎ | ○<br>◎ | ○<br>◎ | ○<br>◎ | ○<br>◎ | ○<br>◎ |
| Example 5 | 0.6 % | 5.0 | ○<br>◎ | ○<br>◎ | ○<br>◎ | ○<br>◎ | ○<br>◎ | ○<br>◎ |
| Example 6 | 0.6 % | 5.0 | ○<br>◎ | ○<br>◎ | ○<br>◎ | ○<br>◎ | ○<br>◎ | ○<br>◎ |
| Example 7 | 0.6 % | 5.5 | ○<br>◎ | ○<br>◎ | ○<br>◎ | ○<br>◎ | ○<br>◎ | ○<br>◎ |
| Example 8 | 0.6 % | 5.5 | ○<br>◎ | ○<br>◎ | ○<br>◎ | ○<br>◎ | ○<br>◎ | ○<br>◎ |
| Reference example 1 | 0 % | 6.0 | | | | | | |
| Reference example 2 | 0.4 % | 6.0 | ○<br>◆ | ×<br>◆ | ×<br>◆ | ×<br>◆ | ×<br>◆ | ×<br>◆ |
| Reference example 3 | 0.6 % | 3.7 | ○<br>◎ | ○<br>◎ | ○<br>◎ | ○<br>◎ | ○<br>◎ | ○<br>◎ |
| Reference example 4 | 0.6 % | 5.5 | ○<br>◆ | ×<br>◆ | ×<br>◆ | ×<br>◆ | ×<br>◆ | ×<br>◆ |
| Reference | 0.6 % | 5.5 | ○ | × | × | × | × | × |

| | | | | | | | | |
|---|---|---|---|---|---|---|---|---|
| example 5 | | | ◆ | ◆ | ◆ | ◆ | ◆ | ◆ |
| Reference example 6 | 0.6 % | 5.5 | ○<br>◆ | ×<br>◆ | ×<br>◆ | ×<br>◆ | ×<br>◆ | ×<br>◆ |
| Reference example 7 | 0.6 % | 5.5 | ○<br>◆ | ×<br>◆ | ×<br>◆ | ×<br>◆ | ×<br>◆ | ×<br>◆ |

Suspension stability of fluticasone furoate

Using Example 7, Reference example 4, Reference example 5, and Reference example 6, the following test was carried out.

(Test method)

The test sample (test preparation) is sufficiently stirred and then the assay sample is taken from the test sample in homogeneous state. The content of fluticasone furoate in the assay sample is determined by high-performance liquid chromatography to give each initial content in homogeneous state (Content A).

Then, 12 g of each test sample in homogeneous state is put into a 13.5 mL glass screw-capped-bottle, and the bottle is well shook again to be in homogeneous state. Separately, the freshly-prepared test sample is centrifuged (5000 rpm, 10 minutes).

Before and after putting the sample into the screw-capped-bottle and shaking it, after 24-hour and one-week leaving to stand, and after centrifuging the sample, the sample in each state is evaluated about the aspect and the content of fluticasone furoate. In determining the content, each test sample is divided into an upper layer (3 g), a middle layer (4 g), and the left lower layer (3 g). Each 2 g of the upper and lower layers is weighed as assay samples, and each content of fluticasone furoate is determined by high-performance liquid chromatography (Content B). The suspension stability is evaluated based on the rate of suspension stability which can be given through the following formula.

$$\text{Rate of suspension stability (\%)} = (\text{Content B})/(\text{Content A}) \times 100$$

(Result: Change in Aspect)

| | | Example 7 | Reference example 4 | Reference example 5 | Reference example 6 |
|---|---|---|---|---|---|
| before shake | upper layer | No phase separation between upper layer and lower layer. A white semitransparent viscous suspension. | No phase separation between upper layer and lower layer. A white opaque suspension. | A semitransparent suspensible liquid | A transparent liquid |
| | lower layer | | | There was a small amount of precipitated crystal at the vessel bottom. | There was a precipitated crystal at the vessel bottom. |

(continued)

| | | Example 7 | Reference example 4 | Reference example 5 | Reference example 6 |
|---|---|---|---|---|---|
| after shake | upper layer | No phase separation between upper layer and lower layer. A white semitransparent viscous suspension. | No phase separation between upper layer and lower layer. A white opaque suspension. | No phase separation between upper layer and lower layer. A white semitransparent viscous suspension. | No phase separation between upper layer and lower layer. A white semitransparent viscous suspension. |
| | lower layer | | | | |
| 24 hours after shake | upper layer | No phase separation between upper layer and lower layer. A white semitransparent viscous suspension. | A white opaque suspensible liquid (which was olopatadine crystal) | A semitransparent suspensible liquid (which was olopatadine crystal) | A semitransparent suspensible liquid (which was olopatadine crystal) |
| | lower layer | | A white opaque suspensible liquid. There was a small amount of precipitate at the vessel bottom (which was olopatadine crystal) | There was a small amount of precipitate at the vessel bottom (which was olopatadine crystal) | There was a precipitated crystal at the vessel bottom (which was olopatadine crystal) |
| one week after shake | upper layer | No phase separation between upper layer and lower layer. A white semitransparent viscous suspension. | A white opaque suspensible liquid (which was olopatadine crystal) | A semitransparent suspensible liquid (which was olopatadine crystal) | A semitransparent suspensible liquid (which was olopatadine crystal) |
| | lower layer | | A white opaque suspensible liquid. There was a precipitate at the vessel bottom (which was olopatadine crystal) | There was a precipitated crystal at the vessel bottom (which was olopatadine crystal) | There was a precipitated crystal at the vessel bottom (which was olopatadine crystal) |
| centrifugation after shake | upper layer | No phase separation between upper layer and lower layer. A white semitransparent viscous suspension. | A white opaque suspensible liquid (which was olopatadine crystal) | A semitransparent suspensible liquid (which was olopatadine crystal) | A semitransparent suspensible liquid (which was olopatadine crystal) |
| | lower layer | | A white opaque suspensible liquid. There was a big amount of precipitate at the vessel bottom (which was olopatadine crystal) | There was a precipitated crystal at the vessel bottom (which was olopatadine crystal) | There was a precipitated crystal at the vessel bottom (which was olopatadine crystal) |

All olopatadine crystals were confirmed to adhere to the instrument wall of the glass screw tube.

(Result: Suspension Stability)

| | | Example 7 | Reference example 4 | Reference example 5 | Reference example 6 |
|---|---|---|---|---|---|
| before shake | upper layer | 99.5 % | 100.3 % | 98.4 % | 97.8 % |
| | lower layer | 99.2 % | 100.9 % | 100.5 % | 100.2 % |
| after shake | upper layer | 99.2 % | 100.7 % | 99.1 % | 97.9 % |
| | lower layer | 100.1 % | 101.9 % | 100.4 % | 98.3 % |
| 24 hours after shake | upper layer | 99.5 % | 101.4 % | 17.8 % | 2.6 % |
| | lower layer | 99.3 % | 101.7 % | 158.9 % | 197.3 % |
| one week after shake | upper layer | 99.2 % | 101.8 % | 15.4 % | 3.3 % |
| | lower layer | 100.3 % | 102.0 % | 199.3 % | 200.7 % |
| centrifugation after shake | upper layer | 99.6 % | 92.9 % | 1.6 % | 0.2 % |
| | lower layer | 99.5 % | 105.7 % | 203.7 % | 201.8 % |

INDUSTRIAL APPLICABILITY

According to the present invention, it becomes possible to prepare a pharmaceutical combination composition comprising a steroid compound and olopatadine free base or a pharmaceutically acceptable salt thereof, especially as a composition for use in nasal administration, wherein olopatadine can be in stable solution state, and the steroid compound can be in a very stable suspension state at an physiologically acceptable pH because the composition has a high viscosity, and the composition does not need to be shaken before use. In addition, the present invention has a good retention in the nasal cavity after spray-administration, and thus it is expected to bring in a sustained and effective improvement of the efficacy.

**Claims**

1. A pharmaceutical composition comprising a steroid compound, and olopatadine or a pharmaceutically acceptable salt thereof.

2. The pharmaceutical composition of claim 1, wherein the olopatadine or a pharmaceutically acceptable salt thereof is olopatadine hydrochloride.

3. The pharmaceutical composition of claim 1 or 2, further comprising carboxy vinyl polymer as a thickening agent.

4. The pharmaceutical composition of any one of claims 1 - 3, comprising a steroid compound, olopatadine or a pharmaceutically acceptable salt thereof, and carboxy vinyl polymer, wherein the pH is adjusted to 4.0 - 7.0.

5. The pharmaceutical composition of any one of claims 1 - 4, wherein the olopatadine or a pharmaceutically acceptable salt thereof is olopatadine hydrochloride, and the olopatadine hydrochloride is in solution state in a concentration of 0.2 % (w/w) or more.

6. The pharmaceutical composition of any one of claims 1 - 5, wherein the steroid compound is in stable dispersion state in a concentration of 0.005 - 1 % (w/w).

7. The pharmaceutical composition of any one of claims 1 - 6, wherein the steroid compound is any one of beclometasone dipropionate, fluticasone propionate, mometasone furoate, or fluticasone furoate.

8. The pharmaceutical composition of claim 7, wherein the steroid compound is fluticasone furoate.

9. The pharmaceutical composition of any one of claims 1 - 8, wherein the concentration of the carboxy vinyl polymer is 0.1 - 2 % (w/w).

10. The pharmaceutical composition of any one of claims 4 - 9, wherein the pH is adjusted with sodium hydroxide and/or hydrochloric acid as a pH adjuster.

11. The pharmaceutical composition of any one of claims 4 - 10, wherein the pH is adjusted with L-arginine as a neutralizing agent.

12. The pharmaceutical composition of any one of claims 1 - 11, further comprising one or more suspension agents.

13. The pharmaceutical composition of claim 12, wherein the suspension agent comprises polysorbate 80.

14. The pharmaceutical composition of any one of claims 1 - 13, further comprising one or more preservatives.

15. The pharmaceutical composition of claim 14, wherein the preservative comprises benzalkonium chloride.

16. The pharmaceutical composition of any one of claims 1 - 15, further comprising one or more stabilizing agents.

17. The pharmaceutical composition of claim 16, wherein the stabilizing agent comprises disodium edetate hydrate.

18. The pharmaceutical composition of any one of claims 1 - 17, further one or more isotonizing agents.

19. The pharmaceutical composition of claim 18, wherein the isotonizing agent comprises sodium chloride and/or glycerin.

20. The pharmaceutical composition of claim 18 or 19, wherein the concentration of the isotonizing agent is 0.1 - 10 % (w/w).

21. The pharmaceutical composition of any one of claims 1 - 20, which is isotonic.

22. The pharmaceutical composition of any one of claims 1 - 21, wherein the viscosity is 250 - 2500 mPa·s.

23. The pharmaceutical composition of any one of claims 1 - 22, whose mean liquid particle size is 30 - 100 μm when sprayed.

24. The pharmaceutical composition of any one of claims 21 - 23, wherein the pH adjuster is sodium hydroxide, the neutralizing agent is L-arginine, the suspension agent is polysorbate 80, the preservative is benzalkonium chloride, the stabilizing agent is disodium edetate hydrate, and the isotonizing agent is glycerin and sodium chloride.

25. The pharmaceutical composition of any one of claims 4 - 24, wherein the pH is adjusted to 4.5 - 6.5.

26. The pharmaceutical composition of any one of claims 4 - 25, wherein the pH is adjusted to 5.0 - 6.0.

27. The pharmaceutical composition of any one of claims 1 - 26, which is an aqueous liquid for use in nasal administration.

28. A formulation for spray-administration to nasal cavity, comprising the pharmaceutical composition of any one of claims 1 - 27.

29. A pharmaceutical composition prepared by adding carboxy vinyl polymer to make olopatadine dissolved at pH of

5.0 - 6.0 and simultaneously make an aqueous suspension comprising fluticasone furoate in a stable suspension state.

## INTERNATIONAL SEARCH REPORT

International application No.
PCT/JP2020/045314

A. CLASSIFICATION OF SUBJECT MATTER
Int.Cl. A61K31/335(2006.01)i, A61K9/10(2006.01)i, A61K9/12(2006.01)i, A61K31/56(2006.01)i, A61K31/573(2006.01)i, A61K31/58(2006.01)i, A61K47/02(2006.01)i, A61K47/10(2006.01)i, A61K47/18(2006.01)i, A61K47/26(2006.01)i, A61K47/32(2006.01)i, A61P11/02(2006.01)i, 61P37/08(2006.01)i, A61P43/00(2006.01)i
FI: A61K31/335, A61K31/573, A61K31/56, A61K31/58, A61P11/02, A61P37/08, A61P43/00121, A61K9/12, A61K9/10, A61K47/32, A61K47/02, A61K47/18, A61K47/26, A61K47/10
According to International Patent Classification (IPC) or to both national classification and IPC

B. FIELDS SEARCHED
Minimum documentation searched (classification system followed by classification symbols)
Int.Cl. A61K31/335, A61K9/10, A61K9/12, A61K31/56, A61K31/573, A61K31/58, A61K47/02, A61K47/10, A61K47/18, A61K47/26, A61K47/32, A61P11/02, A61P37/08, A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580(JDreamIII), CAplus/REGISTRY/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | WO 2011/141929 A2 (CADILA HEALTHCARE LIMITED) 17 November 2011 (2011-11-17), examples 1-3, claims | 1-2, 5, 7-8, 10, 12-17, 18-21, 25-28<br>1-29 |
| X<br>Y | JP 2006-508138 A (AL CONG, INC.) 09 March 2006 (2006-03-09), examples 2-6, claims, paragraph [0006] | 1-2, 5-7, 10, 12-21, 25-28<br>1-29 |
| X<br>Y | JP 2016-534142 A (GRAIN MARK SPECIALTY SA) 04 November 2016 (2016-11-04), examples, claims | 1-2, 5-7, 10, 12-21, 23, 27-28<br>1-29 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 January 2021 | 19 January 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/045314

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2007/123193 A1 (TOKO YAKUHIN KOGYO KABUSHIKI KAISHA) 01 November 2007 (2007-11-01), claims, examples, paragraphs [0036]-[0041], [0043] | 1-29 |
| Y | JP 2-264714 A (TOKO YAKUHIN KOGYO KABUSHIKI KAISHA) 29 October 1990 (1990-10-29), claims, page 4, lower right column, lines 16-20, example 5 | 1-29 |

Form PCT/ISA/210 (second sheet) (January 2015)

**INTERNATIONAL SEARCH REPORT**
Information on patent family members

International application No.
PCT/JP2020/045314

| | | |
|---|---|---|
| WO 2011/141929 A2 | 17 November 2011 | (Family: none) |
| JP 2006-508138 A | 09 March 2006 | US 2004/0097474 A1<br>examples, claims, paragraph [0007]<br>EP 1560586 A1<br>KR 10-2005-0074577 A<br>CN 1711092 A |
| JP 2016-534142 A | 04 November 2016 | US 2015/0079178 A1<br>examples, claims<br>EP 3043773 A1 |
| WO 2007/123193 A1 | 01 November 2007 | US 2009/0275668 A1<br>claims, examples,<br>paragraphs [0062]-[0070]<br>EP 2014305 A1<br>CA 2648553 A<br>KR 10-2009-0005180 A |
| JP 2-264714 A | 29 October 1990 | US 5215739 A<br>claims, column 5,<br>lines 44-51, example 5<br>EP 391342 A1<br>KR 10-1990-0015713 A<br>CN 1046097 A |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4838493 B
- JP 5149308 B
- JP 6203967 B
- WO 2007123193 A
- WO 2007123207 A